# EUROPEAN PATENT APPLICATION

(11) **EP 3 031 896 A1**
(43) Date of publication of application: **15.06.2016**
(21) Application number: 14382512.3
(22) Date of filing: 12.12.2014
(51) Int. Cl.: C12M 1/00, C12M 1/02

(54) **Coupled systems of heat exchange and droplet formation for single-use bioreactors**

(71) Applicant: Universitat Autònoma de Barcelona, 08193 Bellaterra (Cerdanyola del Valles) (ES)
(72) Inventor: Cairó Badillo, Jordi Joan, 08193 Bellaterra (Cerdanyola del Vallès) (ES); Lecina Veciana, Martí, 08193 Bellaterra (Cerdanyola del Vallès) (ES); Casablancas Mira, Antoni, 08193 Bellaterra (Cerdanyola del Vallès) (ES); Martinez Monge, Ivan, 08193 Bellaterra (Cerdanyola del Vallès) (ES); Paredes Muñoz, Carlos J., 08193 Bellaterra (Cerdanyola del Vallès) (ES)

(57) **Abstract**

The present invention relates to an external heat exchange loop coupled to a fluid inlet port equipped with a droplet formation system for the generation of microdrops with diameter sizes ranging from 0,5 microns up to 2 mm, both incorporated to a single-use bioreactor. Both systems will be made of biocompatible plastic or metal material. The former system will allow an optimum energy exchange (heat), while the later will offer better aeration of microorganism's cultures. All these elements described here will be part of a single-use bioreactor (made of biocompatible plastic) for growing different biological specimens (bacteria, yeasts, fungi) at scales of 2 to 2000 liters.

## Description

### Introduction

The use of single use bioreactors (SUB) for animal cell culture at scales between 2 and 2000 liters has been already described (Fenge C, Lüllau E. Cell Culture Biorreactors for Pharmaceutical and Cell Based Therapies. Ozturk S, HU WS, Eds. Taylor and Francis Group: Oxford, UK. 2006; 155-224). The applications of the substances produced through animal cell culture span the fields of biomedicine, biotechnology, food industry and environment. However, there are equally interesting substances produced through the cultivation of bacteria, yeasts and unicellular fungi (mainly aerobic, but also facultative and anaerobic). Unfortunately, currently available SUBs cannot achieve industrially relevant cell densities when using those organisms due their poor removal of the metabolic heat produced by the aerobic metabolism of the main carbon source. If this excess heat is not removed, the optimal process temperature cannot be maintained resulting in poor growth and low product quality and/or titer.

From a production point of view, SUB have the following advantages over stainless steel bioreactors: lower fixed investments in equipment and auxiliary equipment, reduced needs of continuous cycles of cleaning and sterilization and their verification in accordance with GMP standards and, as a consequence, reduced manpower requirements (Sinclaire A, Monge M. Quantitative Economic Evaluation of Single-use Disposables in Bioprocessing. Pharmaceutical Engineering, 2002:20-34). Moreover, the rapid transition between processes enabled by single use systems increases productivity thanks through a reduced turnaround time between batches or even when switching processes.

In addition it has been discovered a surprising effect to achieve industrially relevant cell densities when additionally improving the oxygen transfer rate using a microbubble generating system coupled to an agitation means, independent of it or both integrated.

This invention enables the application of single use bioreactors for the culture of aerobic bacteria, yeasts and fungi by improving both oxygen transfer and heat removal in SUBs. This is done by adding an external broth recirculation loop containing a single use heat exchanger coupled with a droplet/microdrops formation system in the bioreactor.

### Description of the Invention

The bioreactor where this system is implemented is made of a biocompatible plastic material. The single-use bioreactor provides a controlled environment comparable to that of conventional stainless steel bioreactors. The bioreactor is sealed and includes:
- an external recirculation loop with a single-use heat exchanger made of biocompatible plastic,
- a single use droplet/microdrops formation system (percolation) made of plastic for the generation of drops with diameter sizes ranging from 0,5 microns up to 2 mm. This system is located in the path of the returning fluid to the reactor head space
- an agitation shaft,
- a microbubble generation system,
- ports for the introduction, withdrawal and return of fluids (gases and/or liquids),
- ports and/or connectors for the use of equipment for monitoring and controlling the process.

The bioreactor as a whole will be a gamma-sterilized disposable item and will be presented in a sealed envelope or bag. All required handling to operate the bioreactor such as sterile filling, inoculation, connection to/from ports or filters and monitoring elements coupling will not require specific tools. For the final user, the bioreactor will be a consumable item.

### Preferred Description of the Invention

The following describes in more detail the different parts of the apparatus and its operation.

In a preferred embodiment the single use bioreactor has a volume between 2 and 2000 liters.

### Description of external recirculation loop with droplet system-percolation

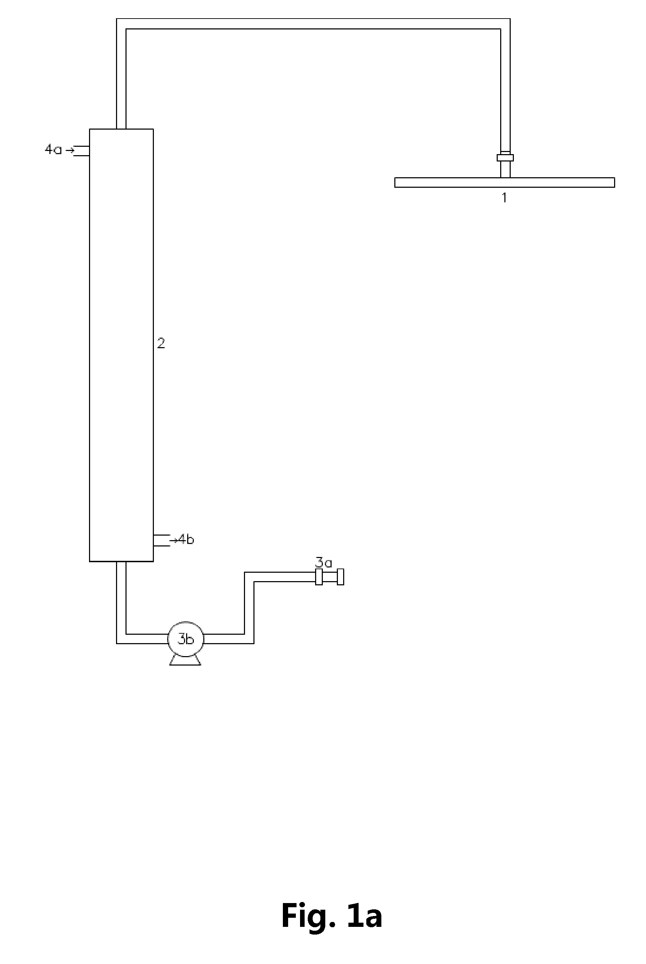

Figure 1a shows a vertical section and plan view of a system using a single use heat exchange system located in the external recirculation loop as previously described.

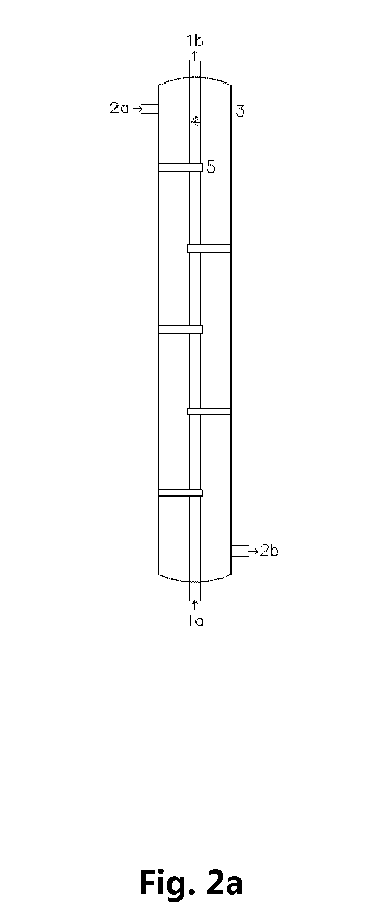

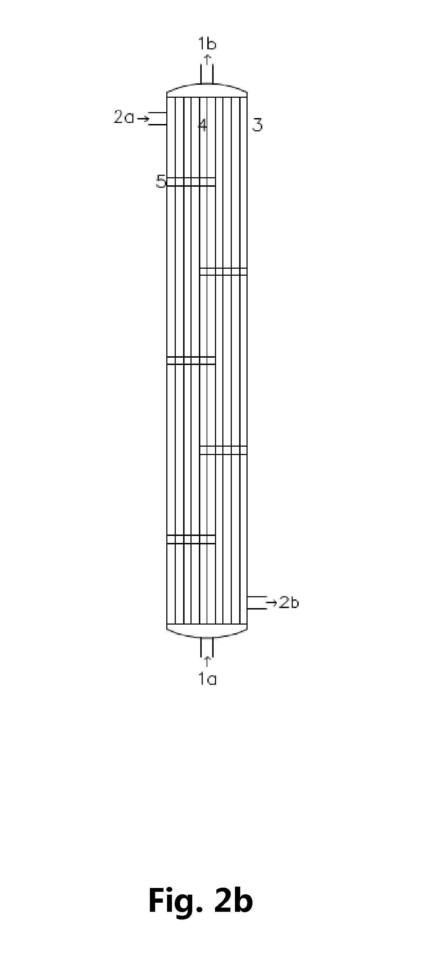

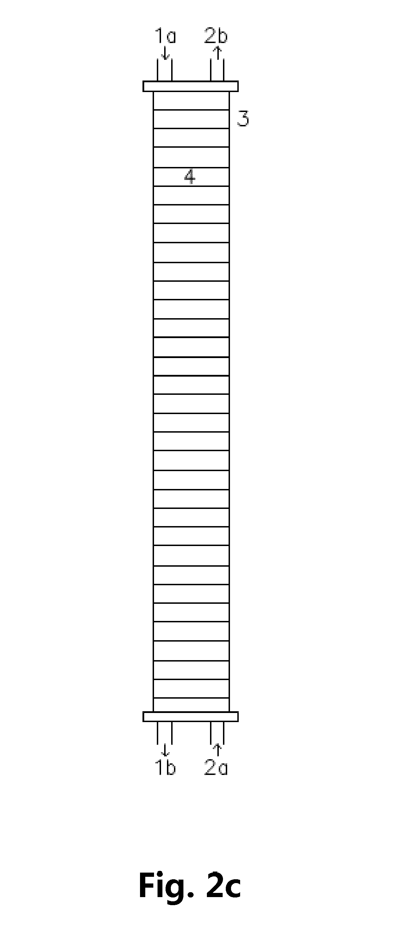

Part of the reactor culture broth is recirculated through a heat exchanger made of biocompatible plastic material (using tubes or plates, figures 2a, 2b and 2c) using a peristaltic pump. After the heat exchanger, the fluid passes through a device which micronizes the fluid into microdrops/droplets with a diameter between 0,5 microns and 2 mm. These microdrops/droplets are then percolated into the reactor. This system improves the transfer of oxygen from the gas mixture to the microdrops.

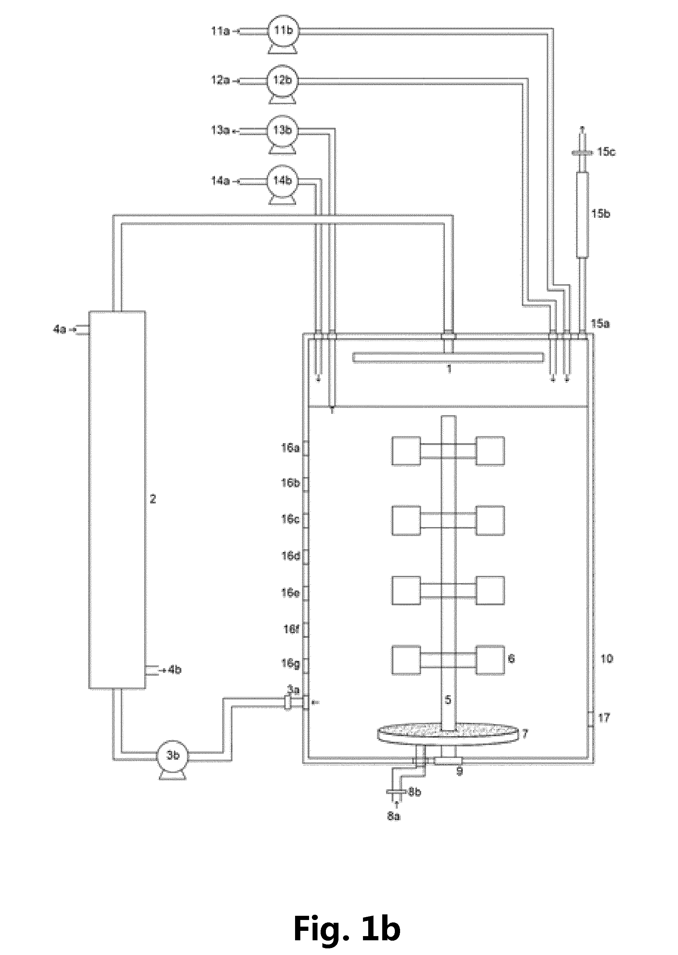

Figure 1b helps to identify the position of the subsystems responsible for the heat exchange in the external recirculation loop, the main body of the bioreactor, the power coupling to the agitation shaft by means of mechanical seal, as well as the ports and access points to the system.

In brief, figures 1a and 1b describe:
1. A closed cylindrical vessel, made of plastic material, whose characteristics can be seen in drawing 1 b. The bioreactor is of single use and, once filled with the culture liquids through a sterile transfer system, can be handled and transported trough a non-sterile environment during the rest of the process
2. A recirculation loop containing a single use system for heat exchange (using tubes or plates, figures 2a, 2b and 2c) as that depicted in drawings 1b. This system is mainly made of plastic.
3. A device situated in the top half of the reactor consisting of a plate of sintered material or one or more layers of a plastic light mesh with micron size openings, or a drilled plate as seen in drawings 3a and 3b. This system micronizes the returning fluid from the recirculation loop resulting in a better heat and oxygen transfer to the generated droplets/microdrops.
4. A system of access points or ports as shown in Figure 1 b. These access points allow the installation of devices that respecting the sterile barrier, enable the automatic monitoring and control of the cultivation parameters.

### Description of the Agitation-Aeration system.

Each device comprises a single central agitation shaft to homogenize its contents moved. This shaft is connected to a mechanical seal located at the bottom or at the top of the bioreactor. The stirring assembly is powered by an electric motor The agitation-aeration element has an agitation shaft driven by an electric motor. This shaft enters the reactor though the top or the bottom of the vessel and has an internal conduit for gases with the gas outlets being sintered metal cylinders for the generation of microbubbles. This sintered piece is included in the stirrer shaft itself and when gas passes through it gas microbubbles are generated. Gas microbubbles can also be generated through a sintered metal plate (plastic or ceramic) at the base of the reactor as shown in Figure 1b.

The bioreactor will also have different inlet ports for gas inflow and outlet ports for exhaust air to control de dissolved oxygen concentration to the culture. These ports will be also used to inflow other gases when necessary. The gas inlet and outlet ports will be equipped with sterile filters (0,22 micrometer) to ensure the sterility of the system.

Additional ports are also present like filling, inoculation, feeding (fed-batch operation), sampling and exhausted media ports.

### Brief Description of the Drawings

For a better understanding of what has been disclosed, we present some drawings that schematically, and in a non-limiting way, exemplify specific embodiments of the invention.
Figure 1a corresponds to the vertical sectional view of the heat exchanger and percolation droplet/microdrops formation systems. Altogether belong to the external recirculation loop, where:
   1 droplet formation system (percolation)
   2 external heat exchange device
   3 a piping
   3 b peristaltic pump
   4 a-b and b cooling-heating piping
   Plan view of an external recirculation loop with a single-use heat exchanger made of biocompatible plastic, a single use droplet/microdrops formation system (percolation) for the generation of microdrops located in the path of the returning fluid to the reactor head space;
Figure 1b belongs to a plan view of respective embodiments of the bioreactor according to the invention, where:
   1 droplet formation system (percolation)
   2 external heat exchange device
   3 a piping
   3 b peristaltic pump
   4 a-b cooling-heating piping
   5 agitation shaft
   6 stirring paddle
   7 microbubble generator
   8a-b gas inlet pipe
   9 mechanical seal
   10 plastic bag bioreactor
   11 a, 12a, 13a, 14a ports for the introduction, withdrawal and return of liquids
   11 b, 12b, 13b, 14b peristaltic pumps
   15 a-b-c gas exhaustion and condenser
   16 ((a-g) optical noninvasive and invasive sensor ports
   17 temperature sensor port
   Plan view of an embodiment showing an external recirculation loop with a single-use heat exchanger made of biocompatible plastic, a single use droplet/microdrops formation system (percolation), an agitation shaft, a microbubble generation system, ports for the introduction, withdrawal and return of fluids (gases and/or liquids), and ports and/or connectors for the use of equipment for monitoring and controlling the process. The agitator shaft, driven by an electric motor, enters from the bottom of the bioreactor;
Figure 1c, 2c and 3c show vertical section views of the heat exchange system;
Figure 2a. detail of the plate heat exchanger, where:
   1 a tube side fluid in
   1b tube side fluid out
   2a cooling-heating fluid in
   2b cooling-heating fluid out
   4 cooling-heating piping
   5 baffles;
Figure 2b. detail of the shell and tube heat exchanger, where:
   1 a tube side fluid in
   1b tube side fluid out
   2a cooling-heating fluid in
   2b cooling-heating fluid out
   3 shell
   4 cooling-heating piping
   5 baffles;
Figure 2c. detail of the plate and frame heat exchanger, where:
   1 a tube side fluid in
   1b tube side fluid out
   2a cooling-heating fluid in
   2b cooling-heating fluid out
   3 shell
   4 plates;

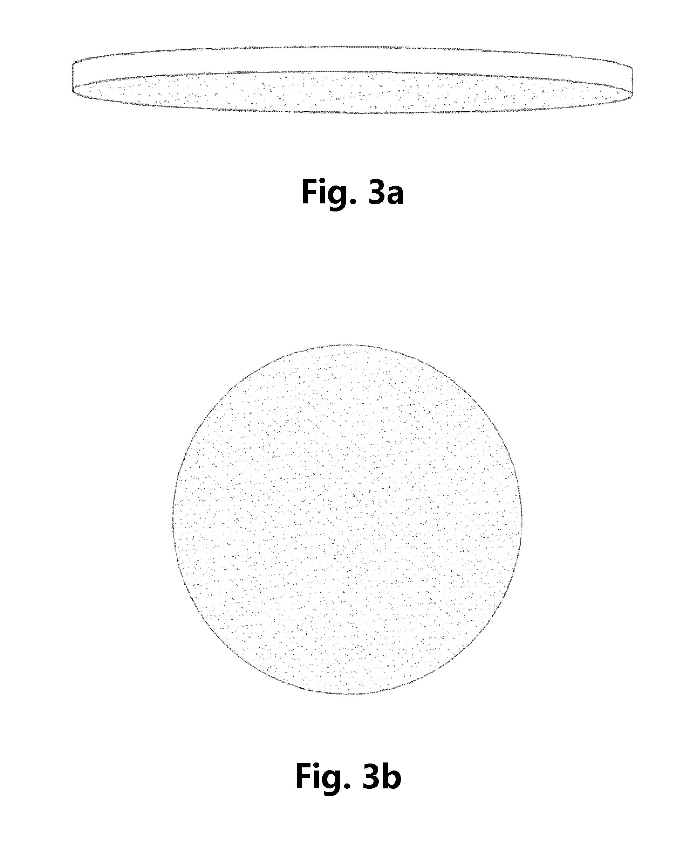

Figure 3a. percolation or droplet/microdrops formation system. Detail of the sintered plate located at the top of the reactor to create microdrops (plan section view);
Figure 3b. percolation or droplet/microdrops formation system. Detail of the sintered plate located at the top of the reactor to create microdrops (vertical section view).

## Claims

1. An external recirculation loop device suitable for a single use bioreactor (SUB) **characterized in that** it comprises:
a) Single-use external heat exchange device.
b) Single-use droplet/microdrops formation device (percolation) located in the path of the returning fluid to the reactor head space.
c) Peristaltic pump.

2. The device of claim 1, wherein a) and b) are made of biocompatible plastic.

3. The device of any of the previous claims, wherein the droplet/microdrops device is suitable for the generation of microdrops with diameter sizes ranging from 0,5 microns up to 2 mm.

4. The device of any of the previous claims, further comprising a device suitable for improving the oxygen transfer **characterized in that** it comprises :
a) Microbubble generation system, and
b) Agitation means

5. Single use bioreactor **characterized in that** it comprises a device according any of the claims 1-4
